# EUROPÄISCHE PATENTSCHRIFT

(11) **EP 4 039 830 B1**
(45) Veröffentlichungstag und Bekanntmachung des Hinweises auf die Patenterteilung: **09.09.2026**
(21) Anmeldenummer: 22158383.4
(22) Anmeldetag: 11.12.2014
(51) Int. Cl.: C21B 5/06, C21B 7/00, C21C 5/38, C25B 1/04, C07C 29/151

(54) **ANLAGENVERBUND ZUR STAHLERZEUGUNG UND VERFAHREN ZUM BETREIBEN DES ANLAGENVERBUNDES**
PLANT COMBINATION FOR PRODUCING STEEL AND METHOD FOR OPERATING THE PLANT COMBINATION
INSTALLATIONS INTERCONNECTÉES DESTINÉES À LA PRODUCTION D'ACIER ET LEUR PROCÉDÉ DE FONCTIONNEMENT

(30) Priorität: 12.12.2013 DE 102013113913
(43) Veröffentlichungstag der Anmeldung: 10.08.2022
(62) Dokumentnummer(n) der früheren Anmeldung(en) nach Art. 76 EPÜ: 14815582.3 / 3 080 309
(73) Patentinhaber: thyssenkrupp AG, 45143 Essen (DE)
(72) Erfinder: Achatz, Reinhold, 45259 Essen (DE); Wagner, Jens, 60439 Frankfurt a.M. (DE); Oles, Markus, 45527 Hattingen (DE); Schmöle, Peter, 44141 Dortmund (DE); Kleinschmidt, Ralph, 45472 Mülheim a.d.Ruhr (DE); Kolbe, Bärbel, 58452 Witten (DE); Krüger, Matthias, 44625 Herne (DE); Meißner, Christoph, 44534 Lünen (DE)
(74) Vertreter: thyssenkrupp Intellectual Property GmbH

(56) Entgegenhaltungen:
- DE-A1- 102010 049 639
- US-A1- 2006 027 043
- HAMID GHANBARI ET AL: "Optimal design and operation of a steel plant integrated with a polygeneration system", AICHE JOURNAL, vol. 59, no. 10, 1 October 2013 (2013-10-01), US, pages 3659 - 3670, XP055501257, ISSN: 0001-1541, DOI: 10.1002/aic.14098
- SCHMOELE P ET AL: "ECOLOGICAL HOT METAL PRODUCTION USING COKE PLANT AND BLAST FURNACE ROUTE//PRODUCTION ECOLOGIQUE DE FONTE PAR LA FILIERE COKERIE ET HAUT-FOURNEAU", REVUE DE METALLURGIE - CAHIERS D'INFORMATIONS TECHNIQUES, REVUE DE METALLURGIE. PARIS, FR, vol. 102, no. 3, 1 March 2005 (2005-03-01), pages 171 - 182, XP001230940, ISSN: 0035-1563, DOI: 10.1051/METAL:2005140

## Beschreibung

Die Erfindung betrifft einen Anlagenverbund zur Stahlerzeugung.

Der Anlagenverbund zur Stahlerzeugung umfasst einen Hochofen zur Roheisenerzeugung, ein Konverterstahlwerk zur Rohstahlerzeugung, ein Gasleitungssystem für Gase, die bei der Roheisenerzeugung und/oder der Rohstahlerzeugung anfallen, sowie ein Kraftwerk zur Stromerzeugung. Das Kraftwerk ist als Gasturbinenkraftwerk oder Gasturbinen- und Dampfturbinenkraftwerk ausgelegt und wird mit einem Gas betrieben, welches zumindest eine Teilmenge des bei der Roheisenerzeugung im Hochofen anfallenden Hochofengichtgases und/oder eine Teilmenge des in dem Konverterstahlwerk anfallenden Konvertergases umfasst.

Im Hochofen wird aus Eisenerzen, Zuschlägen sowie Koks und anderen Reduktionsmitteln wie Kohle, Öl, Gas, Biomassen, aufbereiteten Altkunststoffen oder sonstigen Kohlenstoff und/oder Wasserstoff enthaltenden Stoffen Roheisen gewonnen. Als Produkte der Reduktionsreaktionen entstehen zwangsläufig CO, CO₂, Wasserstoff und Wasserdampf. Ein aus dem Hochofenprozess abgezogenes Hochofengichtgas weist neben den vorgenannten Bestandteile häufig einen hohen Gehalt an Stickstoff auf. Die Gasmenge und die Zusammensetzung des Hochofengichtgases ist abhängig von den Einsatzstoffen und der Betriebsweise und unterliegt Schwankungen. Typischerweise enthält Hochofengichtgas jedoch 35 bis 60 Vol.-% N₂, 20 bis 30 Vol.-% CO, 20 bis 30 Vol.-% CO₂ und 2 bis 15 Vol.-% H₂. Rund 30 bis 40% des bei der Roheisenerzeugung entstehenden Hochofengichtgases wird im Regelfall zum Aufheizen des Heißwindes für den Hochofenprozess in Winderhitzern eingesetzt; die verbleibende Gichtgasmenge kann in anderen Werksbereichen auch extern zu Heizzwecken oder zur Stromerzeugung genutzt werden.

Im Konverterstahlwerk, das dem Hochofenprozess nachgeschaltet ist, wird Roheisen zu Rohstahl umgewandelt. Durch Aufblasen von Sauerstoff auf flüssiges Roheisen werden störende Verunreinigungen wie Kohlenstoff, Silizium, Schwefel und Phosphor entfernt. Da die Oxidationsprozesse eine starke Wärmeentwicklung verursachen, wird häufig Schrott in Mengen bis zu 25% bezogen auf das Roheisen als Kühlmittel zugesetzt. Ferner werden Kalk zur Schlackenbildung und Legierungsmittel zugegeben. Aus dem Stahlkonverter wird ein Konvertergas abgezogen, welches einen hohen Gehalt an CO aufweist und ferner Stickstoff, Wasserstoff und CO₂ enthält. Eine typische Konvertergaszusammensetzung weist 50 bis 70 Vol.-% CO, 10 bis 20 Vol.-% N₂, ca. 15 Vol.-% CO₂ und ca. 2 Vol.-% H₂ auf. Das Konvertergas wird entweder abgefackelt oder bei modernen Stahlwerken aufgefangen und einer energetischen Nutzung zugeführt.

Der Anlagenverbund kann optional im Verbund mit einer Kokerei betrieben werden. In diesem Fall umfasst der eingangs beschriebene Anlagenverbund zusätzlich eine Koksofenanlage, in der Kohle durch einen Verkokungsprozess in Koks umgewandelt wird. Bei der Verkokung von Kohle zu Koks fällt ein Koksofengas an, welches einen hohen Wasserstoffgehalt und beachtliche Mengen an CH₄ enthält. Typischerweise enthält Koksofengas 55 bis 70 Vol.-% H₂, 20 bis 30 Vol.-% CH₄, 5 bis 10 Vol.-% N₂ und 5 bis 10 Vol.-% CO. Zusätzlich weist das Koksofengas Anteile von CO₂, NH₃ und H₂S auf. In der Praxis wird das Koksofengas in verschiedenen Werksbereichen zu Heizzwecken und im Kraftwerksprozess zur Stromerzeugung genutzt. Darüber hinaus ist es bekannt, Koksofengas zusammen mit Hochofengichtgas oder mit Konvertergas zur Erzeugung von Synthesegasen zu verwenden. Gemäß einem aus WO 2010/136313 A1 bekannten Verfahren wird Koksofengas aufgetrennt in einen wasserstoffreichen Gasstrom und einen CH₄ und CO enthaltenen Restgasstrom, wobei der Restgasstrom dem Hochofenprozess zugeführt wird und der wasserstoffreiche Gasstrom mit Hochofengichtgas gemischt und zu einem Synthesegas weiterverarbeitet wird. Aus EP 0 200 880 A2 ist es bekannt, Konvertergas und Koksofengas zu mischen und als Synthesegas für eine Methanolsynthese zu nutzen. Weitere Beispiele sind aus HAMID GHANBARI ET AL: "Optimal design and operation of a steel plant integrated with a polygeneration system", American Institute of Chemical Engineers, AI CH E JOURNAL, Bd. 59, Nr. 10, 1. Oktober 2013, Seiten 3629-3670, SCHMOELE ET AL: "Ecological Hot Metal Production Using Coke Plant and Blast Furnace Route", Revue de Metallurgie - Cahiers d'Informations Techniques, Bd. 102, Nr. 3, Seiten 171-182, 1.März 2005, sowie US 2006/027043A1 bekannt.

In einem integrierten Hüttenwerk, welches im Verbund mit einer Kokerei betrieben wird, werden etwa 40 bis 50 % der als Hochofengichtgas, Konvertergas und Koksofengas anfallenden Rohgase für verfahrenstechnische Prozesse eingesetzt. Etwa 50 bis 60 % der entstehenden Gase werden dem Kraftwerk zugeführt und zur Stromerzeugung genutzt. Der im Kraftwerk erzeugte Strom deckt den Strombedarf für die Roheisen- und Rohstahlerzeugung. Im Idealfall ist die Energiebilanz geschlossen, so dass abgesehen von Eisenerzen und Kohlenstoff in Form von Kohle und Koks als Energieträger kein weiterer Eintrag von Energie notwendig ist und außer Rohstahl und Schlacke kein Produkt den Anlagenverbund verlässt.

Vor diesem Hintergrund liegt der Erfindung die Aufgabe zugrunde, die Wirtschaftlichkeit des Gesamtprozesses weiter zu verbessern und einen Anlagenverbund anzugeben, mit dem es möglich ist, die Kosten für die Stahlerzeugung zu reduzieren.

Ausgehend von einem Anlagenverbund zur Stahlerzeugung mit einem Hochofen zur Roheisenerzeugung, einem Konverterstahlwerk zur Rohstahlerzeugung, einem Gasleitungssystem für Gase, die bei der Roheisenerzeugung und/ oder der Rohstahlerzeugung anfallen, und einem Kraftwerk zur Stromerzeugung ist erfindungsgemäß eine Biotechnologieanlage vorgesehen, die an das Gasleitungssystem angeschlossen ist und hinsichtlich der Gasversorgung parallel zu dem Kraftwerk geschaltet ist. Das Gasleitungssystem umfasst erfindungsgemäß eine betrieblich steuerbare Gasweiche zur Aufteilung der dem Kraftwerk und der Biotechnologieanlage zugeführten Gasmengenströme. Vorteilhafte Ausgestaltungen des erfindungsgemäßen Anlagenverbundes werden in den Ansprüchen 2 bis 5 beschrieben.

Zur Deckung des Strombedarfes des Anlagenverbundes wird extern bezogener Strom und Kraftwerkstrom, der von dem Kraftwerk des Anlagenverbundes erzeugt wird, herangezogen. Dabei wird der Stromanteil des extern bezogenen Stroms bezogen auf den gesamten Strombedarf des Anlagenverbundes als variable Prozessgröße festgelegt und wird die dem Kraftwerksprozess zugeführte Nutzgasmenge in Abhängigkeit dieser Prozessgröße festgelegt. Der nicht zur Stromerzeugung genutzte Teil des Nutzgases wird nach einer Gaskonditionierung einer biotechnologischen Anlage zugeführt und für biochemische Prozesse genutzt.

In einer nicht erfindungsgemäßen Chemieanlage können chemische Produkte aus Synthesegasen erzeugt werden, welche jeweils die Komponenten des Endproduktes enthalten. Chemische Produkte können beispielsweise Ammoniak oder Methanol oder auch andere Kohlenwasserstoffverbindungen sein.

Zur Herstellung von Ammoniak muss ein Synthesegas bereitgestellt werden, welches Stickstoff und Wasserstoff im richtigen Verhältnis enthält. Der Stickstoff kann aus Hochofengichtgas gewonnen werden. Als Wasserstoffquelle kann Hochofengichtgas oder Konvertergas verwendet werden, wobei Wasserstoff durch Konvertierung des CO-Anteils durch eine Wasser-Gas-Shift-Reaktion (CO + H₂O 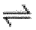 CO₂ + H₂) erzeugt wird. Zur Herstellung von Kohlenwasserstoffverbindungen, beispielsweise Methanol, muss ein im Wesentlichen aus CO und/oder CO₂ und -H₂ bestehendes Synthesegas bereitgestellt werden, welches die Komponenten Kohlenmonoxid und/oder Kohlendioxid und Wasserstoff im richtigen Verhältnis enthält. Das Verhältnis wird häufig durch das Modul (H₂ - CO₂) / (CO + CO₂) beschrieben. Der Wasserstoff kann beispielsweise durch Konvertierung des CO-Anteils im Hochofengichtgas durch eine Wasser-Gas-Shift-Reaktion erzeugt werden. Zur Bereitstellung von CO kann Konvertergas herangezogen werden. Als CO₂-Quelle kann Hochofengichtgas und/oder Konvertergas dienen.

Anstelle einer nicht erfindungsgemäßen Chemieanlage zur Erzeugung von Produkten aus Synthesegas wird im Rahmen der Erfindung eine biotechnologische Anlage eingesetzt. Hierbei handelt es sich um eine Anlage zur Fermentation von Synthesegas. Das Synthesegas wird über eine Fermentation biochemisch genutzt, wobei Produkte wie Alkohole (Ethanol, Butanol), Aceton oder organische Säuren hergestellt werden können. Auch diese Produkte, die durch Fermentation von Synthesegas erzeugt werden, sind im vorliegenden Fall nur beispielhaft genannt.

Gemäß einer bevorzugten Ausführung der Erfindung umfasst der Anlagenverbund zusätzlich eine Koksofenanlage. Wenn die Roheisenerzeugung und die Rohstahlerzeugung im Verbund mit einer Kokerei betrieben wird, kann eine Teilmenge des bei der Roheisenerzeugung anfallenden Hochofengichtgases und/oder eine Teilmenge des im Konverterstahlwerk angefallenen Konvertergases mit einer Teilmenge des in der Koksofenanlage entstehenden Koksofengases gemischt werden und das Mischgas als Nutzgas verwendet werden. Zur Erzeugung eines Synthesegases beispielsweise für die Ammoniaksynthese kann als Rohgas eine Mischung aus Koksofengas und Hochofengichtgas oder ein Mischgas aus Koksofengas, Konvertergas und Hochofengichtgas verwendet werden. Zur Herstellung von Kohlenwasserstoffverbindungen eignet sich ein Mischgas aus Koksofengas und Konvertergas oder ein Mischgas aus Koksofengas, Konvertergas und Hochofengichtgas.

Die Rohgase - Koksofengas, Konvertergas und/oder Hochofengichtgas - können einzeln oder in Kombinationen als Mischgas werden. Die Aufbereitung insbesondere von Kokosofengas umfasst eine Gasreinigung zur Abtrennung störender Inhaltsstoffe, insbesondere Teer, Schwefel und Schwefelverbindungen, aromatischen Kohlenwasserstoffen (BTX) und hochsiedenden Kohlenwasserstoffen. Zum Herstellen des Synthesegases ist ferner eine Gaskonditionierung notwendig. Im Rahmen der Gaskonditionierung wird der Anteil der Komponenten CO, CO₂, H₂ innerhalb des Rohgases verändert. Die Gaskonditionierung umfasst beispielsweise eine Druckwechseladsorption zur Abtrennung und Anreicherung von H₂ und/oder eine Wasser-Gas-Shift-Reaktion zur Umwandlung von CO in Wasserstoff und/oder einen Steam-Reformer zur Umwandlung des CH₄-Anteils in CO und Wasserstoff im Koksofengas.

Zumindest eine Teilmenge des bei der Roheisenerzeugung im Hochofen anfallenden Hochofengichtgases und/ oder eine Teilmenge des in dem Konverterstahlwerk anfallenden Konvertergases wird als Rohgas genutzt, um daraus durch biochemische Prozesse in einer biotechnologischen Anlage Produkte, das heißt Wertstoffe, zu erzeugen. Gemäß einer bevorzugten Ausführung der Erfindung wird die Anlage im Verbund mit einer Kokerei betrieben und wird Koksofengas in die Nutzung einbezogen. Als Konsequenz aus der Nutzung eines Teils dieser Gase fehlt dem Anlagenverbund Strom, der extern bezogen werden muss. Der extern bezogene Strom kann aus konventionellen Kraftwerken stammen oder aus erneuerbaren Energien gewonnen werden. Vorzugsweise wird der extern bezogene Strom vollständig oder zumindest teilweise aus erneuerbarer Energie gewonnen und stammt beispielsweise aus Windkraftanlagen, Solaranlagen, geothermischen Kraftwerken, Wasserkraftwerken, Gezeitenkraftwerken und dergleichen. Zur Erreichung eines möglichst wirtschaftlichen Betriebs des Anlagenverbunds wird Strom in Zeiten niedriger Strompreise zugekauft und zur Versorgung des Anlagenverbunds verwendet und wird der nicht zur Stromerzeugung genutzte Teil des Nutzgases nach einer Gaskonditionierung in einer biotechnologischen Anlage zur Herstellung von chemischen Produkten verwendet. In Zeiten hoher Strompreise wird das Nutzgas hingegen dem Kraftwerk vollständig oder zumindest zu einem großen Teil zugeführt, um Strom zur Versorgung des Anlagenverbundes zu erzeugen. Die biotechnologische Anlage wird in Zeiten hoher Strompreise entsprechend heruntergefahren. Zum Betrieb des Verfahrens wird eine Regelung vorgesehen, die den wechselseitigen Betrieb des Kraftwerkes einerseits und der biotechnologischen Anlage andererseits in Abhängigkeit einer variablen Prozessgröße festgelegt. Die Prozessgröße wird vorzugsweise in Abhängigkeit einer Funktion bestimmt, welche den Preis für den extern bezogenen Strom und die Kosten für die Erzeugung des Kraftwerkstroms als Variablen enthält.

Das Verfahren ermöglicht einen wirtschaftlichen Betrieb des Anlagenverbundes. Dabei nutzt das Verfahren insbesondere auch aus, dass der Wirkungsgrad eines Kraftwerksprozess zur Erzeugung von Strom schlechter ist als der Wirkungsgrad einer biotechnologischen Anlage, in der durch biochemische Prozesse aus Synthesegas Chemieprodukte hergestellt werden.

Die Leistung des Kraftwerkes kann in Abhängigkeit der dem Kraftwerksprozess zugeführten Nutzgasmenge zwischen 20 % und 100 % geregelt werden. Als Kraftwerk wird vorzugsweise ein Gasturbinenkraftwerk oder ein Gas- und Dampfturbinenkraftwerk verwendet.

Die Leistung der biotechnologischen Anlage wird in Abhängigkeit der diesen Anlagen zugeführten Mischgasmenge geregelt. Eine wesentliche Herausforderung für eine nicht erfindungsgemäße Chemieanlage ist die dynamische Fahrweise bei wechselnden Anlagenlasten.

Die Verwendung einer biotechnologischen Anlage hat den Vorteil, dass eine biotechnologische Anlage flexibler hinsichtlich Lastwechsel ist als eine Chemieanlage.

Im Folgenden wird die Erfindung anhand einer lediglich ein Ausführungsbeispiel darstellenden Zeichnung erläutert. Es zeigen schematisch
- Fig. 1: ein stark vereinfachtes Blockschaltbild eines Anlagenverbundes zur Stahlerzeugung mit einem Hochofen zur Roheisenerzeugung und einem Konverterstahlwerk zur Rohstahlerzeugung, einem Kraftwerk und einer Biotechnologieanlage,
- Fig. 2: das stark vereinfachte Blockschaltbild eines Anlagenverbundes, der zusätzlich zu einem Hochofen zur Roheisenerzeugung und einem Konverterstahlwerk zur Rohstahlerzeugung einem Kraftwerk und einer Biotechnologieanlage auch eine Koksofenanlage umfasst,
- Fig. 3: das Blockschaltbild eines Anlagenverbundes entsprechend Fig. 2 mit einer zusätzlichen Anlage zur Wasserstofferzeugung.

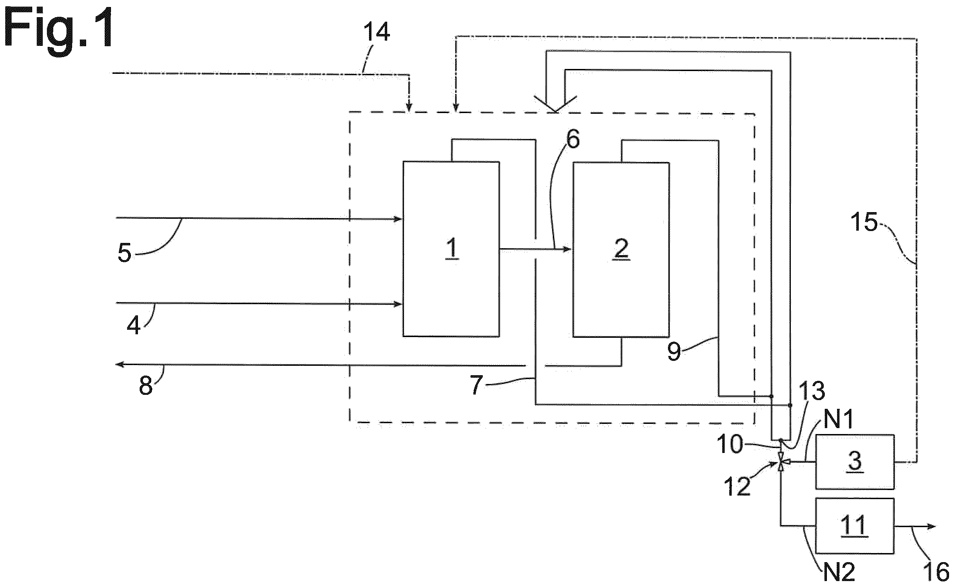

Der in Fig. 1 dargestellte Anlagenverbund zur Stahlerzeugung umfasst einen Hochofen 1 zur Roheisenerzeugung, ein Konverterstahlwerk 2 zur Rohstahlerzeugung, ein Kraftwerk 3 zur Stromerzeugung und eine Biotechnologieanlage 11.

Im Hochofen 1 wird im Wesentlichen aus Eisenerz 4 und Reduktionsmitteln 5, insbesondere Koks und Kohle, Roheisen 6 gewonnen. Durch Reduktionsreaktionen entsteht ein Hochofengichtgas 7, welches als Hauptbestandteile Stickstoff, CO, CO₂ und H₂ enthält. Im Konverterstahlwerk 2, das dem Hochofenprozess nachgeschaltet ist, wird Roheisen 6 zu Rohstahl 8 umgewandelt. Durch Aufblasen von Sauerstoff auf das flüssige Roheisen werden störende Verunreinigungen, insbesondere Kohlenstoff, Silizium und Phosphor entfernt. Zur Kühlung kann Schrott in Mengen bis zu 25 % bezogen auf die Roheisenmenge zugeführt werden. Ferner werden Kalk zur Schlackenbildung und Legierungsmittel zugegeben. Am Kopf des Konverters wird ein Konvertergas 9 abgezogen, welches einen sehr hohen Anteil an CO aufweist.

Das Kraftwerk 3 ist als Gasturbinenkraftwerk oder Gasturbinen- und Dampfturbinenkraftwerk ausgelegt und wird mit einem Gas betrieben, welches zumindest eine Teilmenge des bei der Roheisenerzeugung im Hochofen 1 anfallenden Hochofengichtgases 7 und eine Teilmenge des im Konverterstahlwerk 2 anfallenden Konvertergases 9 umfasst. Zur Führung der Gase ist ein Gasleitungssystem vorgesehen.

Gemäß einer in Fig. 1 dargestellten Gesamtbilanz wird dem Anlagenverbund Kohlenstoff als Reduktionsmittel 5 in Form von Kohle und Koks sowie Eisenerz 4 zugeführt. Als Produkte fallen Rohstahl 8 und Rohgase 7, 9 an, die sich in Menge, Zusammensetzung, Heizwert und Reinheit unterscheiden und an verschiedenen Stellen im Anlagenverbund wieder eingesetzt werden. Bei einer Gesamtbetrachtung wird 40 bis 50 %, zumeist etwa 45 %, der Rohgase 7, 9 wieder in den Hüttenprozess zur Roheisenerzeugung oder Rohstahlerzeugung zurückgeführt. Zwischen 50 und 60 %, zumeist etwa 55 %, der Rohgase 7, 9 kann zum Betrieb des Kraftwerkes 3 genutzt werden. Das mit einem Mischgas 10 aus Hochofengichtgas 7 und Konvertergas 9 betriebene Kraftwerk 3 wird so ausgelegt, dass es den Strombedarf des Anlagenverbundes decken kann.

Gemäß der Darstellung in Fig. 1 ist eine Biotechnologieanlage 11 vorgesehen, die an das Gasleitungssystem angeschlossen ist und hinsichtlich der Gasversorgung parallel zu dem Kraftwerk 3 geschaltet ist. Das Gasleitungssystem weist eine betrieblich steuerbare Gasweiche 12 zur Aufteilung der dem Kraftwerk 3 und der Biotechnologieanlage 11 zugeführten Gasmengeströme auf. In Strömungsrichtung vor der Gasweiche ist eine Mischvorrichtung 13 zur Herstellung des aus Hochofengichtgas 7 und Konvertergas 9 bestehenden Mischgases 10 vorgesehen.

Bei dem in Fig. 1 dargestellten Anlagenverbund wird zumindest eine Teilmenge des bei der Roheisenerzeugung im Hochofen 1 anfallenden Hochofengichtgases 7 und eine Teilmenge des bei der Rohstahlerzeugung anfallenden Konvertergases 9 als Nutzgas zum Betrieb des Kraftwerkes 3 und der Biotechnologieanlage 11 verwendet. Zur Deckung des Strombedarfes des Anlageverbundes wird extern bezogener Strom 14 und Kraftwerkstrom 15, der von dem Kraftwerk 3 des Anlagenverbundes erzeugt wird, herangezogen. Der Stromanteil des extern bezogenen Stroms 14 bezogen auf den gesamten Strombedarf des Anlagenverbundes wird als variable Prozessgröße festgelegt und die dem Kraftwerk 3 zugeführte Nutzgasmenge N1 wird in Abhängigkeit dieser Prozessgröße bestimmt. Der nicht zur Stromerzeugung genutzte Teil des Nutzgases N2 wird nach einer Gaskonditionierung als Synthesegas zur Herstellung chemischer Produkte 16 der biotechnologischen Anlage zugeführt und für biochemische Prozesse genutzt.

Der extern bezogene Strom 14 wird vorzugsweise vollständig oder zumindest teilweise aus erneuerbarer Energie gewonnen und stammt beispielsweise aus Windkraftanlagen, Solaranlagen, Wasserkraftwerken und dergleichen. Die Prozessgröße, auf deren Grundlage die dem Kraftwerksprozess zugeführte Nutzgasmenge N1 festgelegt wird, wird in Abhängigkeit einer Funktion bestimmt, welche den Preis für den extern bezogenen Strom und die Kosten für die Erzeugung des Kraftwerkstromes 15 als Variablen enthält. Zur Erreichung eines möglichst wirtschaftlichen Betriebs des Anlagenverbundes wird Strom in Zeiten niedriger Strompreise als externer Strom 14 zugekauft und zur Stromversorgung des Anlagenverbundes verwendet, wobei der nicht zur Stromerzeugung genutzte Teil des Nutzgases N2 der Biotechnologieanlage 11 zugeführt und nach einer Gaskonditionierung als Synthesegas zur Herstellung chemischer Produkte 16 verwendet wird. In Zeiten hoher Strompreise werden die bei der Roheisenerzeugung und Rohstahlerzeugung anfallenden Rohgase 7, 9 dem Kraftwerk 3 zugeführt, um den Strom zur Versorgung des Anlagenverbundes zu erzeugen. Die biotechnologische Anlage wird in Zeiten hoher Strompreise entsprechend heruntergefahren.

Die Leistung des Kraftwerkes 3 wird in Abhängigkeit der dem Kraftwerkprozess zugeführten Nutzgasmenge N1 zwischen 20 % und 100 % geregelt. Die Leistung der biotechnologischen Anlage wird in Abhängigkeit der dieser Anlage zugeführten Nutzgasmenge N2 geregelt.

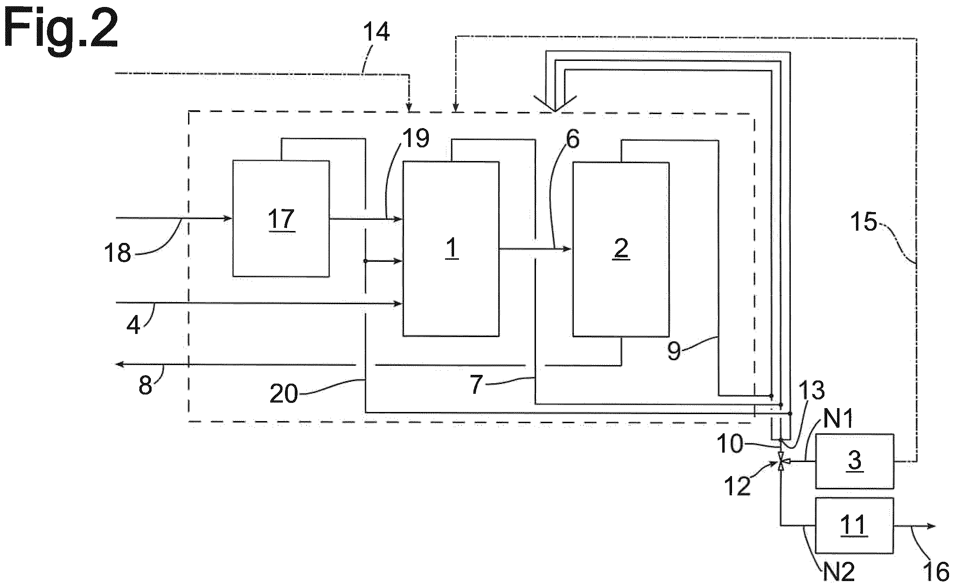

Im Ausführungsbeispiel der Fig. 2 umfasst der Anlagenverbund zusätzlich eine Koksofenanlage 17. Bei der Verkokung von Kohle 18 zu Koks 19 fällt Koksofengas 20 an, welches einen hohen Anteil an Wasserstoff und CH₄ enthält. Teile des Koksofengases 20 können für die Beheizung der Winderhitzer im Hochofen 1 genutzt werden. Das Gasleitungsystem schließt eine Gasverteilung für das Koksofengas 20 ein. In Strömungsrichtung vor der Gasweiche 12 ist eine Mischvorrichtung 13 zur Herstellung eines aus Hochofengichtgas 7, Konvertergas 9 und Koksofengas 20 bestehenden Mischgases 10 vorgesehen. Mit der Gasweiche sind die dem Kraftwerk 3 und der Biotechnologieanlage 11 zugeführten Gasmengenströme steuerbar.

Beim Betrieb der in Fig. 2 dargestellten Anlage wird eine Teilmenge des bei der Roheisenerzeugung anfallenden Hochofengichtgases 7 und/oder eine Teilmenge des im Konverterstahlwerk anfallenden Konvertergases 9 mit einer Teilmenge des in der Koksofenanlage 17 entstehenden Koksofengases 20 gemischt. Das Mischgas 10 wird als Nutzgas zum Betrieb des Kraftwerkes 3 und der biotechnologischen Anlage verwendet.

Das Hochofengichtgas 7, das Konvertergas 9 und das Koksofengas 20 können beliebig miteinander kombiniert werden. Die Kombination der Gasströme 7, 9, 20 richtet sich nach dem gewünschten Synthesegas bzw. dem Produkt, welches in der biotechnologischen Anlage unter Verwendung des Synthesegases hergestellt werden soll.

Im Rahmen der Erfindung ist es beispielsweise möglich, dass Hochofengichtgas 7 und Konvertergas 9 gemischt werden, dass aus dem Mischgas nach einer Gaskonditionierung ein Synthesegas hergestellt wird und dass dem Synthesegas oder dem gereinigten Mischgas vor der Weiterverarbeitung zu dem Synthesegas zusätzlich aufbereitetes Koksofengas 20 zugemischt wird.

Ferner besteht die Möglichkeit, dass aus Hochofengichtgas 7 nach einer Gaskonditionierung ein Synthesegas hergestellt wird und dass dem Synthesegas oder dem gereinigten Hochofengichtgas vor der Weiterverarbeitung zu dem Synthesegas zusätzlich aufbereitetes Koksofengas 20 zugemischt wird.

Schließlich besteht die Möglichkeit, dass aus Konvertergas 9 nach einer Gaskonditionierung ein Synthesegas hergestellt wird und dass dem Synthesegas oder dem gereinigten Konvertergas vor der Weiterverarbeitung zu dem Synthesegas zusätzlich aufbereitetes Koksofengas 20 zugemischt wird.

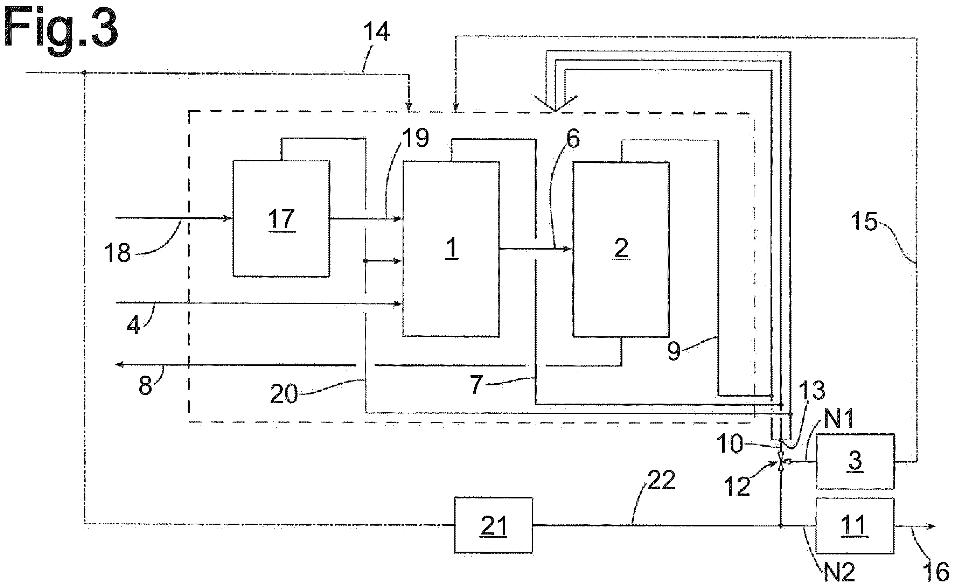

Bei den in den Fig. 1 und 2 dargestellten Betriebsweise kann der Kohlenstoffgehalt und der Stickstoffgehalt der beim Betrieb des Anlageverbundes anfallenden Rohgase nicht vollständig zur Herstellung von chemischen Produkten genutzt werden, da ein Wasserstoffunterschuss vorliegt. Um den Kohlenstoffgehalt und den Stickstoffgehalt des Nutzgases vollständig für die Herstellung von chemischen Wertstoffen einzusetzen, weist der in Fig. 3 dargestellte Anlagenverbund zusätzlich eine Anlage 21 zur Wasserstofferzeugung auf, die durch eine wasserstoffführende Leitung 22 mit dem Gasleitungssystem verbunden ist. Die Anlage 21 zur Wasserstofferzeugung kann insbesondere eine Elektrolyseanlage zur Wasserelektrolyse sein. Der Betrieb einer Wasserelektrolyse ist energieintensiv und wird daher primär in Zeiten niedriger Strompreise in Betrieb genommen, zu denen auch die Biotechnologieanlage betrieben wird und das Kraftwerk 3 heruntergefahren ist. Der zusätzlich erzeugte Wasserstoff wird der biotechnologischen Anlage zusammen mit dem Mischgas zugeführt. Dadurch kann die Kapazität der biotechnologischen Anlage deutlich gesteigert werden.

## Patentansprüche

1. Anlagenverbund zur Stahlerzeugung mit
einem Hochofen (1) zur Roheisenerzeugung,
einem Konverterstahlwerk (2) zur Rohstahlerzeugung,
einem Gasleitungssystem für Gase, die bei der Roheisenerzeugung und/oder der Rohstahlerzeugung anfallen,
einem Kraftwerk (3) zur Stromerzeugung,
wobei das Kraftwerk (3) als Gasturbinenkraftwerk oder Gasturbinen- und Dampfturbinenkraftwerk ausgelegt ist und mit einem Gas betrieben wird, welches zumindest eine Teilmenge des bei der Roheisenerzeugung im Hochofen anfallenden Hochofengichtgases (7) und/oder eine Teilmenge des in dem Konverterstahlwerk (2) anfallenden Konvertergases (9) umfasst,
**dadurch gekennzeichnet, dass** eine Biotechnologieanlage (11) für biochemische Fermentations-Prozesse vorgesehen ist, die an das Gasleitungssystem angeschlossen ist und hinsichtlich der Gasversorgung parallel zu dem Kraftwerk (3) geschaltet ist, und dass das Gasleitungssystem eine betrieblich steuerbare Gasweiche (12) zur Aufteilung der dem Kraftwerk (3) und der Biotechnologieanlage (11) zugeführten Gasmengenströme umfasst,
wobei eine Regelung vorgesehen ist, die dazu konfiguriert ist, den wechselseitigen Betrieb des Kraftwerkes einerseits und der Biotechnologieanlage andererseits in Abhängigkeit einer variablen Prozessgröße festzulegen, wobei die Prozessgröße in Abhängigkeit einer Funktion bestimmt wird, welche den Preis für extern bezogenen Strom und Kosten für die Erzeugung von Kraftwerkstrom als Variablen enthält.

2. Anlagenverbund nach Anspruch 1, **dadurch gekennzeichnet, dass** der Anlagenverbund zusätzlich eine Koksofenanlage (17) umfasst und dass das Gasleitungssystem eine Gasverteilung für Koksofengas (20), welches bei einem Verkokungsprozess in der Koksofenanlage (17) anfällt, einschließt.

3. Anlagenverbund nach Anspruch 1 oder 2, **dadurch gekennzeichnet, dass** das Gasleitungssystem in Strömungsrichtung vor der Gasweiche (12) eine Mischvorrichtung (13) zur Herstellung eines aus Hochofengichtgas (7) und/oder Konvertergas (9) und/oder Koksofengas (20) bestehenden Mischgases (10) aufweist und dass mittels der Gasweiche (12) die dem Kraftwerk (3) und der Chemie- oder Biotechnologieanlage (11) zugeführten Gasmengenströme steuerbar sind.

4. Anlagenverbund nach einem der Ansprüche 1 bis 3, **dadurch gekennzeichnet, dass** der Anlagenverbund zusätzlich eine Anlage (21) zur Wasserstofferzeugung aufweist, die durch eine Wasserstoff führende Leitung (22) mit dem Gasleitungssystem verbunden ist.

5. Anlagenverbund nach Anspruch 4, **dadurch gekennzeichnet, dass** die Anlage (21) zur Wasserstofferzeugung eine Elektrolyseanlage zur Wasserelektrolyse ist.

## Claims

1. Plant complex for steel production comprising a blast furnace (1) for pig iron production, a converter steelworks (2) for crude steel production, a gas pipeline system for gases generated during pig iron production and/or crude steel production, a power plant (3) for electricity generation, wherein the power plant (3) is designed as a gas turbine power plant or a gas turbine and steam turbine power plant and is operated with a gas which comprises at least a partial quantity of the blast furnace top gas (7) generated during pig iron production in the blast furnace and/or at least a partial quantity of the converter gas (9) generated in the converter steelworks (2), **characterized in that** a biotechnology plant (11) for biochemical fermentation processes is provided, which is connected to the gas pipeline system and is connected in parallel with the power plant (3) with respect to the gas supply, and **in that** the gas pipeline system comprises an operationally controllable gas diverter (12) for dividing the gas volume flows supplied to the power plant (3) and the biotechnology plant (11), wherein a control system is provided which is configured to determine the coordinated operation of the power plant on the one hand and the biotechnology plant on the other hand as a function of a variable process parameter, wherein the process parameter is determined based on a function which contains the price of externally procured electricity and costs for the generation of power plant electricity as variables.

2. Plant complex according to claim 1, **characterized in that** the plant complex additionally comprises a coke oven plant (17) and **in that** the gas pipeline system includes a gas distribution for coke oven gas (20), which is generated during a coking process in the coke oven plant (17).

3. Plant complex according to claim 1 or 2, **characterized in that** the gas pipeline system has, in the flow direction upstream of the gas diverter (12), a mixing device (13) for producing a mixed gas (10) consisting of blast furnace top gas (7) and/or converter gas (9) and/or coke oven gas (20), and **in that** the gas volume flows supplied to the power plant (3) and the chemical or biotechnology plant (11) are controllable by means of the gas diverter (12).

4. Plant complex according to one of claims 1 to 3, **characterized in that** the plant complex additionally has a plant (21) for hydrogen production, which is connected to the gas pipeline system by a hydrogen-carrying line (22).

5. Plant complex according to claim 4, **characterized in that** the plant (21) for hydrogen production is an electrolysis plant for water electrolysis.

## Revendications

1. Ensemble d'installation pour la production d'acier comprenant un haut fourneau (1) pour la production de fonte brute, une aciérie à convertisseur (2) pour la production d'acier brut, un système de conduites de gaz pour des gaz produits lors de la production de fonte brute et/ou de la production d'acier brut, une centrale électrique (3) pour la production d'électricité, la centrale électrique (3) étant conçue comme une centrale à turbine à gaz ou une centrale à turbine à gaz et à turbine à vapeur et étant exploitée avec un gaz qui comprend au moins une partie du gaz de gueulard de haut fourneau (7) produit lors de la production de fonte brute dans le haut fourneau et/ou au moins une partie du gaz de convertisseur (9) produit dans l'aciérie à convertisseur (2), **caractérisé en ce qu'**une installation de biotechnologie (11) pour des processus de fermentation biochimique est prévue, laquelle est raccordée au système de conduites de gaz et est montée en parallèle avec la centrale électrique (3) en ce qui concerne l'alimentation en gaz, et **en ce que** le système de conduites de gaz comprend un aiguillage de gaz (12) commandable en fonctionnement pour la répartition des débits de gaz alimentés vers la centrale électrique (3) et l'installation de biotechnologie (11),
une régulation étant prévue, laquelle est configurée pour déterminer le fonctionnement alterné de la centrale électrique d'une part et de l'installation de biotechnologie d'autre part en fonction d'une variable de procédé variable, la variable de procédé étant déterminée en fonction d'une fonction qui contient comme variables le prix de l'électricité achetée à l'extérieur et les coûts de la production d'électricité de la centrale électrique.

2. Ensemble d'installation selon la revendication 1, **caractérisé en ce que** l'ensemble d'installation comprend en outre une installation de cokerie (17) et **en ce que** le système de conduites de gaz comprend une distribution de gaz pour le gaz de cokerie (20), lequel est produit lors d'un processus de cokéfaction dans l'installation de cokerie (17).

3. Ensemble d'installation selon la revendication 1 ou 2, **caractérisé en ce que** le système de conduites de gaz présente, dans le sens d'écoulement en amont de l'aiguillage de gaz (12), un dispositif de mélange (13) pour la production d'un gaz mélangé (10) constitué de gaz de gueulard de haut fourneau (7) et/ou de gaz de convertisseur (9) et/ou de gaz de cokerie (20), et **en ce que** les débits de gaz alimentés vers la centrale électrique (3) et l'installation de chimie ou de biotechnologie (11) sont commandables au moyen de l'aiguillage de gaz (12).

4. Ensemble d'installation selon l'une des revendications 1 à 3, **caractérisé en ce que** l'ensemble d'installation présente en outre une installation (21) pour la production d'hydrogène, laquelle est reliée au système de conduites de gaz par une conduite transportant de l'hydrogène (22).

5. Ensemble d'installation selon la revendication 4, **caractérisé en ce que** l'installation (21) pour la production d'hydrogène est une installation d'électrolyse pour l'électrolyse de l'eau.
